(19) Europäisches Patentamt
European Patent Office
Office européen des brevets

(11) Publication number: **0 259 530 B1**

(12) **EUROPEAN PATENT SPECIFICATION**

(45) Date of publication of patent specification: **08.04.92** (51) Int. Cl.$^5$: **C07D 249/20**

(21) Application number: **86307057.9**

(22) Date of filing: **12.09.86**

(54) **Method for preparing 2-phenylbenzotriazoles and 2-phenylbenzotriazole-N-oxides.**

(43) Date of publication of application:
**16.03.88 Bulletin 88/11**

(45) Publication of the grant of the patent:
**08.04.92 Bulletin 92/15**

(84) Designated Contracting States:
**CH DE FR GB IT LI**

(56) References cited:

**CHEMICAL ABSTRACTS, vol. 98, no. 15, 11th April 1983, page 643, abstract no. 126102b, Columbus, Ohio, US**

**CHEMICAL ABSTRACTS, vol. 106, no. 7, 16th February 1987, page 647, abstract no. 50230c, Columbus, Ohio, US**

(73) Proprietor: **CHEMIPRO KASEI KAISHA, LTD.
No. 2-15, Gokodori 5-chome Chuo-ku
Kobe-shi Hyogo-ken(JP)**

(72) Inventor: **Seino, Shuichi
No. 3-6, Karibadai 5-chome Nishi-ku
Kobe-shi Hyogo-ken(JP)**

(74) Representative: **Perry, Robert Edward et al
GILL JENNINGS & EVERY 53-64 Chancery Lane
London WC2A 1HN(GB)**

**EP 0 259 530 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for preparing 2-phenylbenzotriazoles and 2-phenylbenzotriazole-N-oxides.

2-Phenylbenzotriazoles of formula I, wherein $R_1$ is H, Cl, $C_{1-4}$ alkyl, $C_{1-4}$ alkoxy, COOH or $SO_3H$; $R_2$ is H, Cl, $C_{1-4}$ alkyl or $C_{1-4}$ alkoxy; $R_3$ is H, Cl, $C_{1-12}$ alkyl, $C_{1-4}$ alkoxy. phenyl, ($C_{1-8}$ alkyl)phenyl, phenoxy or phenyl($C_{1-4}$ alkyl); $R_4$ is H, Cl, OH or $C_{1-4}$ alkoxy; and $R_5$ is H, $C_{1-12}$ alkyl or phenyl ($C_{1-4}$ alkyl), are known, and have utility as ultra-violet absorbers which can be added to, say, plastics, paints and oils. 2-Phenylbenzotriazole-N-oxides of formula II, wherein $R_1$, $R_2$, $R_3$, $R_4$ and $R_5$ are as defined above, are valuable intermediates in the preparation of the 2-phenylbenzotriazoles (I).

These known compounds have previously been produced by chemically or electrolytically reducing nitroazobenzenes of formula III, where $R_1$, $R_2$, $R_3$, $R_4$ and $R_5$ are as defined above. However, the known methods are not always satisfactory.

JP-A-37/5934 and US-A-3773751 disclose chemically reducing nitroazobenzenes (III) in an alcoholic sodium hyroxide solution with zinc powder. The yield is satisfactory but zinc sludge is produced, causing waste water contamination.

US-A-2362988 discloses chemical reduction of nitroazobenzenes (III) using ammonium sulphide, an alkali sulphide, zinc-ammonia, hydrogen sulphide-sodium or zinc-hydrochloric acid. This method produces a large amount of sulphite or zinc salts, thus contaminating waste water. Further, sulphites generates sulphurous acid gas, and the sulphide reducing agents generate poisonous hydrogen sulphide.

JP-A-51138679 and JP-A-51138680 disclose reduction using hydrogen under pressure. JP-A-5088072 discloses reduction using hydrazine. These methods give poor yields and are not economic. Further, it is impossible to obtain the desired product in high purity, owing to side-reactions. For example, when producing a chlorine-containing product, dechlorination occurs.

C.A. 98, 126102b (= JP-A-57167976) and C.A. 106, 50230c (= JP-A-611975700 disclose the conversions III→II→I and III→I, the reactions taking place in the presence of an aromatic ketone, a base and an aldehyde reducing agent.

According to a first aspect of the present invention, a method for preparing a 2-phenylbenzotriazole (I) as defined above comprises reducing a nitroazobenzene (III) as defined above with an aldehyde in the presence of base and an aromatic ketone selected from anthrone, benzanthrone and 9-fluorenone.

According to a second aspect of the invention, a method for preparing a 2-phenylbenzotriazole (I) as defined above comprises reducing 2-phenylbenzotriazole-N-oxide (II) as defined above with, per mol thereof, 1 to 4 mol aldehyde in the presence of base and an aromatic ketone as defined above.

According to a third aspect of the invention, a method for preparing a 2-phenylbenzotriazole-N-oxide (II) as defined above comprises reducing nitroazobenzene (III) as defined above with, per mol thereof, 1 to 2 mol aldehyde in the presence of base and an aromatic ketone as defined above.

The processes of the invention give technically and economically satisfactory results, without causing environmental pollution. The desired product is produced in higher purity, and has better thermostability, than before.

The method according to the first aspect of the invention, i.e. IIITI, can be carried out in either one or two steps, depending on the conditions such as temperature and the amount of the aldehyde which is used. For a one-step reaction, a suitable temperature is 20 to 130 ° C, preferably 40 to 100 ° C, 2 to 4 mols of the aldehyde reducing agent being used per mol of the starting material (III).

The two-step method is sometimes advantageous in terms of product purity and yield, despite the use of two steps. In the first step, 1 to 2 mol aldehyde are preferably used per mol starting material (III); the temperature is suitably 40 to 100 C. In the second step, 1 to 4 mol, preferably 1 to 2 mol, aldehyde are preferably used per mol of the resulting intermediate (II). The given preferred temperatures are also preferred for the independent III→I and II→I methods according to the second and third aspects of the invention.

In order that the III→I, III→II or II→I process should proceed smoothly, it is preferably conducted in aqueous solution, or in an inert solvent such as an alcohol, toluene, acetone, dimethyl sulphoxide or acetonitrile; a mixture of the inert solvent with water may also be used. If necessary or desired, a surface-active agent or a phase-transfer catalyst may also be used.

Examples of nitroazobenzenes of formula III are given in European Patent Application No. 86306514.0, among which are:

2-nitro-2′-hydroxy-5′-methylazobenzene,
2-nitro-2′-hydroxy-5′-t-octylazobenzene,
2-nitro-2′-hydroxy-5′-t-butylazobenzene,

2-nitro-2′,4′-dihydroxyazobenzene,
2-nitro-4-chloro-2′-4′-dihydroxyazobenzene,
2-nitro-2′-hydroxy-4′-methylazobenzene,
2-nitro-2′-hydroxy-5′-t-amylazobenzene,
2-nitro-4′-chloro-2′-hydroxy-5′-t-amylazobenzene,
2-nitro-2′-hydroxy-5′-n-dodecylazobenzene,
2-nitro-4-chloro-2′-hydroxy-5′-n-dodecylazobenzene,
2-nitro-4-chloro-2′-hydroxy-5′-t-octylazobenzene,
2-nitro-4-methyl-2′-hydroxy-5′-methylazobenzene,
2-nitro-4,6-dichloro-2′-hydroxy-5′-t-butylazobenzene, and
2-nitro-4-carboxy-2′-hydroxy-5-methylazobenzene.

Examples of 2-phenylbenzotriazole-N-oxides of formula II are given in European Patent Application No. 86306514.0; those and others are:

2-(2-hydroxy-5-methylphenyl)benzotriazole-N-oxide,
2-(2-hydroxy-5-t-butylphenyl)benzotriazole-N-oxide,
2-(2-hydroxy-5-t-octylphenyl)benzotriazole-N-oxide,
2-(2,4-dihydroxyphenyl)benzotriazole-N-oxide,
2-(2,4′-dihydroxyphenyl)-5-chlorobenzotriazole-N-oxide,
2-(2-hydroxy-4′-methoxyphenyl)benzotriazole-N-oxide,
2-(2-hydroxy-5-t-amylphenyl)benzotriazole-N-oxide,
2-(2-hydroxy-5-n-dodecylphenyl)benzotriazole-N-oxide,
2-(2-hydroxy-5-n-dodecylphenyl)-5-chlorobenzotriazole-N-oxide,
2-(2-hydroxy-5-t-octylphenyl)-5-chlorobenzotriazole-N-oxide,
2-(2-hydroxy-5-methylphenyl)-5-methylbenzotriazole-N-oxide,
2-(2-hydroxy-5-methylphenyl)-5-carboxybenzotriazole-N-oxide,
2-(2-hydroxy-3-t-butyl-5-methylphenyl)-5-chlorobenzotriazole-N-oxide,
2-(2-hydroxy-3,5-di-t-butylphenyl)-5-chlorobenzotriazole-N-oxide,
2-(2-hydroxy-3,5-di-t-amylphenyl)benzotriazole-N-oxide,
2-(2-hydroxy-3,5′-di-t-butylphenyl)benzotriazole-N-oxide,
2-(2-hydroxy-3-t-butyl-5-methylphenyl)benzotriazole-N-oxide,
2-(2-hydroxy-3,5′-di-t-octylphenyl)benzotriazole-N-oxide,
2-(2-hydroxy-3,5′-di-t-octylphenyl)-5-chlorobenzotriazole-N-oxide,
2-(2-hydroxy-3-t-butyl-5-methylphenyl)-5-methylbenzotriazole-N-oxide,
2-(2-hydroxy-3,5,-di-t-butylphenyl)-5-n-butylbenzo-triazole-N-oxide,
2-(2-hydroxy-3-sec-butyl-5-t-butylphenyl)-5-n-butylbenzo-triazole-N-oxide,
2-(2-hydroxy-3-sec-butyl-5-t-butylphenyl)-5-t-butylbenzo-triazole-N-oxide,
2-(2-hydroxy-3,5-di-t-butylphenyl)-5,7-dichlorobenzotri-azole-N-oxide
2-(2-hydroxy-3,5-di-t-amylphenyl)-5-chlorobenzotriazole--N-oxide,
2-[2-hydroxy-3,5-di-($\alpha$,$\alpha$-dimethylbenzyl)phenyl]benzotriazole-N-oxide,
2-[2-hydroxy-3,5-di-($\alpha$,$\alpha$–dimethylbenzyl)phenyl] -5-chloro-benzotriazole-N-oxide,
2-(2-hydroxy-$\alpha$-methylbenzyl-5-methylphenyl)benzotriazoleN-oxide, and
2-(2-hydroxy-$\alpha$-methylbenzyl-5-methylphenyl)chlorobenzotriazole-N-oxide.

The 2-phenylbenzotriazole-N-oxides (II) are preferably prepared by reduction of the nitroazobenzenes (III). Alternatively, for use in the II→I conversion according to the invention, they may be obtained by any other suitable process.

The nitroazobenzenes (III) may be obtained by a diazotisation reaction between a nitroaniline of formula IV and a phenol of formula V.

The aldehyde reducing agent is preferably of the formula RCHO, wherein R is H, alkyl, phenyl or substituted phenyl. Examples are formaldehyde, paraformaldehyde, acetoaldehyde, benzaldehyde and anisaldehyde. The first two of these are most preferred.

The aromatic ketone is anthrone, 9-fluorenone or benzanthrone. Two or more ketones may be used in admixture, and this is sometimes preferred.

The aromatic ketone is used generally in an amount of 0.2 to 30%, preferably 2 to 20%, by weight based on the weight of the starting material, i.e. nitroazobenzene (III) or 2-phenylbenzotriazole-N-oxide (II).

The base used in the present invention is, for example, sodium hydroxide or potassium hydroxide. Such a base is preferably used in an amount of 1 to 12 mol, more preferably 2 to 8 mol, per mol of the starting material, i.e. nitroazobenzene (III) or 2-phenylbenzotriazole-N-oxide (II).

The Present invention is further illustrated by the following Examples.

Example 1

2-nitro-2'-hydroxy-5'-methylazobenzene (12.9 g) was added to a mixture of methanol (60 ml), water (30 ml) and 97 % sodium hydroxide (12,4 g) and the resultant mixture was stirred while raising temperature to 65 ° C. Thereafter, the mixture was cooled to 40 ° C, and 9-fluorenone (0.8 g) and then 80 % paraformaldehyde (2.1 g) were added to the mixture for 1 hour. The resultant mixture was then heated to the boiling point (75 ° C), and Was stirred at the boiling point for 6 hours, thus almost all of the azobenzene having disappeared to produce 2-(2-hydroxy-5-methylphenyl)benzotriazole-N-oxide.

Thereafter, water (50 ml) was added to the reaction liquor, and the reaction liquor was neutralized to pH 8 with 62 % sulfuric acid (16 g) to precipitate a crystal. The crystal thus obtained was filtered by suction, and the separated crystal was then dried, thus producing 10.7 g of 2-(2-hydroxy-5-methylphenyl) benzotriazole-N-oxide having a melting point of 138 to 140 ° C at the yield of 89.0 %.

Example 2

The same procedure as in Example 1 was repeated, except that 2-nitro-2'-hydroxy-5'-methylazobenzene (12.9 g) was replaced respectively by (a) 2-nitro-2'-hydroxy-5' -butylazobenzene (14.9 g) (b) 2-nitro-2'-hydroxy-5'-t-octylazobenzene (17.8 g), and (c) 2-nitro-2',4'-dihydroxy-azobenzene (14.9 g).

The products thus obtained and their properties are as follows:

(a) 2-(2-hydroxy-5-t-butylphenyl)benzotriazole-N-oxide:
Yield: 12.7 g (90.0 %), Melting Point: 73 to 78 ° C
(b) 2-(2-hydroxy-5-t-octylphenyl)benzotriazole-N-oxide:
Yield: 14.2 g (84.0 %), Melting Point: 106 to 111 ° C
(c) 2-(2,4'-dihydroxyphenyl)benzotriazole-N-oxide:
Yield: 9.7 g (80.0 %), Melting Point: 246 to 248 ° C

Example 3

95 % potassium hydroxide (17.7 g) was dissolved in a mixture of ethyl alcohol (80 ml) and water (10 ml). 2-nitro-2'-hydroxy-5'-methylazobenzene (12.9 g) was then added to the resultant solution at 50 to 60 ° C for 30 minutes while stirring, and thereafter 9-fluorenone (0.6 g) and 35 % formalin (5.6 g) were added to the solution in 30 minutes. The resultant mixture was stirred for 1 hour at 65 to 70 ° C, and was further reacted at the boiling point (85 ° C) for 4 hours, thus almost all of the azobenzene having disappeared to produce 2-(2-hydroxy-5-methylphenyl)benzotriazole-N-oxide.

Thereafter, water (50 ml) was added to the reaction liquor, and the reaction liquor was neutralized to pH 8 with 62 % sulfuric acid to precipitate a crystal. The crystal thus obtained was filtered by suction to separate the crystal, and the separated crystal was fully washed with water and further with ethyl alcohol. The washed crystal was then dried, thus producing 10.6 g of 2-(2-hydroxy-5-methylphenyl)benzotriazole-N-oxide having a melting point of 138 to 140 ° C at the yield of 88.0 %.

Example 4

Methanol (60 ml), water (30 ml), 97% sodium hydroxide (12.4 g), and 2-nitro-2'-hydroxy-5'-methylazobenzene (12.9 g) were mixed and stirred. After adding benzanthrone (1.2 g) to the resultant mixture at 65 to 70 ° C, 80 % paraformaldehyde (2.3 g) was added to the mixture for 2 hours, and then the reaction liquor was further stirred at the boiling point (75 ° C) for 5 hours; o-nitroazobenzene disappeared, to produce 2-(2-hydroxy-5-methyl-phenyl)benzotriazole-N-oxide.

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with methanol. The washed crystal was then dried, thus producing 10.1 g of 2-(2-hydroxy-5-methylphenyl)benzotriazole-N-oxide having a melting point of 138 to 140 ° C at the yield of 83.5 %.

Example 5

2-nitro-2'-hydroxy-5'-methylazobenzene (12.9 g) was added to a mixture of methanol (60 ml), water (30 ml) and 97 % sodium hydroxide (12.4 g), and the resultant mixture was stirred while raising temperature to 65 ° C. 9-fluorenone (1.2 g) and then 90 % acetoaldehyde (3.2 g) were added to the mixture for 1 hour. The

resultant mixture was then heated to the boiling point (75° C), and was stirred at the boiling point (75° C) for 6 hours, thus almost all of the azobenzenes having disappeared to produce 2-(2-hydroxy-5-methylphenyl)-benzotriazole-N-oxide.

Thereafter, water (50 ml) was added to the reaction liquor, and the reaction liquor was neutralized to pH 8 with 62 % sulfuric acid (16 g) to precipitate a crystal. The crystal thus obtained was filtered by suction, and the separated crystal was fully washed with water and further with methanol. The washed crystal was then dried, thus producing 10.5 g of 2-(2-hydroxy-5-methylphenyl)-benzotriazole-N-oxide having a melting point of 138 to 140° C at the yield of 87.0 %.

Example 6

2-nitro-2′-hydroxy-5′-methylazobenzene (12.9 g) was added to a mixture of methanol (60 ml), water (30 ml) and 97 % sodium hydroxide (12.4 g), and the resultant mixture was stirred while raising temperature to 65° C. Thereafter, the mixture was cooled to 40° C , and 9-fluorenone (0.8 g) and then 80 % paraformaldehyde (2.1 g) were added to the mixture for 1 hour. The resultant mixture was then heated to the boiling point (75° C), and was stirred at the boiling point for 6 hours, thus almost all of the azobenzene having disappeared to produce 2-(2-hydroxy-5-methylphenyl)benzotriazole-N-oxide (Process(b) having completed).

In order to conduct Process (c), the reaction liquor was then cooled to 70° C, 9-fluorenone (0.2 g) and then 80 % paraformaldehyde (2.5 g) were added to the reaction liquor for 30 minutes. Thereafter, the reaction liquor was heated to the boiling point (75° C), and was stirred for futher 4 hours, thus the N-oxide having disappeared to complete Process (c).

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with methanol. The washed crystal was then dried, thus producing 9.2 g of 2-(2-hydroxy-5-methylphenyl)benzotriazole having a melting point of 128 to 130° C at the yield of 81.8 %.

Example 7

The same procedure as in Example 6 was repeated, except that 2-nitro-2′-hydroxy-5′-methylazobenzene (12.9 g) was replaced respectively by (a) 2-nitro-2′-hydroxy-5′-butylazobenzene (14.9 g), (b) 2-nitro-2′-hydroxy-5′-t-octylazobenzene (17.8 g), and (c) 2-nitro-2′,4′-dihydroxy-azobenzene (14.9 g).

The product thus obtained and their properties are as follows:

(a) 2-(2-hydroxy-5-t-butylphenyl)benzotriazole:
Yield: 11.0 g (84.0 %), Melting Point: 95.5 to 98.0° C
(b) 2-(2-hydroxy-5-t-octylphenyl)benzotriazole:
Yield: 12.8 g (79.0 %), Melting Point: 103 to 105° C
(c) 2-(2,4-dihydroxyphenyl)benzotriazole:
Yield: 9.2 g (70.5 %), Melting Point: 187 to 189° C

Example 8

95 % potassium hydroxide (17.7 g) was dissolved in a mixture of ethyl alcohol (80 ml) and water (10 ml). 2-nitro-2′-hydroxy-5′-methylazobenzene (12.9 g) was then added to the resultant solution at 50 to 60° C for 30 minutes while stirring, and thereafter 9-fluorenone (0.6 g) and 35 % formalin (5.6 g) were added to the solution in 30 minutes. The resultant mixture was stirred for 1 hour at 65 to 70° C, and was further reacted at the boiling point (85° C) for 4 hours, thus almost all of the azobenzene having disappeared to complete the reaction of Process (b). Anthrone (0.4 g) and 35 % formalin (6.4 g) were then added to the reaction liquor by dropwise for 30 minutes, and the reaction liquor was further reacted for 8 hours while stirring, thus the intermediate product of Process (b) having disappeared to complete the reaction of Process (c).

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with ethyl alcohol. The washed crystal was then dried, thus producing 8.7 g of 2-(2-hydroxy-5-methylphenyl)benzotriazole-N-oxide having a melting point of 138 to 140° C at the yield of 77.3 %.

Example 9

Methanol (60 ml), water (30 ml), 97% sodium hydroxide (12.4 g), and 2-nitro-2′-hydroxy-5′-methylazobenzene (12.9 g) were mixed and stirred. After adding benzanthrone (1.2 g) to the resultant mixture at 65 to 70 ° C, 80 % paraformaldehyde (4.6 g) was added to the mixture for 2 hours, and then the reaction liquor was further stirred at the boiling point (75 ° C) for 5 hours; o-nitroazobenzene disappeared, to produce 2-(2-hydroxy-5-methylphenyl)benzotriazole-N-oxide (Process (b) having completed). In order to conduct Process (c), the reaction liquor was further stirred for 6 hours, thus the N-oxide having disappeared to complete Process (c).

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with methanol. The washed crystal was then dried, thus producing 8.4 g of 2-(2-hydroxy-5-methylphenyl)benzotriazole having a melting point of 128 to 130 ° C at the yield of 74.7 %.

Example 10

2-nitro-2′-hydroxy-5′-methylazobenzene (12.9 g) was added to a mixture of methanol (60 ml), water (30 ml) and 97 % sodium hydroxide (12.4 g), and the resultant mixture was stirred while raising temperature to 65 ° C. 9-fluorenone (1.2 g) and then 90 % acetoaldehyde (6.5 g) were added to the mixture for 1 hour. The resultant mixture was then stirred at the boiling point (75 ° C) for 6 hours, thus almost all of the azobenzene having disappeared to produce 2-(2-hydroxy-5-methylphenyl)-benzotriazole-N-oxide (Process(b) having completed). In order to conduct Process (c), the reaction liquor was further stirred at the boiling point for 5 hours, thus the N-oxide having disappeared to complete Process (c).

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with methanol. The washed crystal was then dried, thus producing 9.1 g of 2-(2-hydroxy-5-methylphenyl)benzotriazole having a melting point of 128 to 130 ° C at the yield of 80.9 %.

Example 11

Methanol (110 ml), water (20 ml), 97% sodium hydroxide (12.4 g) and 2-nitro-2′-hydroxy-5′-t-octylazobenzene (17.8 g) were mixed and stirred. After adding 9-fluorenone (2.4 g) to the resultant mixture at 65 to 70 ° C, 80 % paraformaldehyde (6 g) was added to the mixture for 4 hours, and then the reaction liquor was further stirred at the boiling point (73 ° C) for 6 hours, thus the reduction reaction having completed.

Thereafter, water (50 ml) was added to the reaction liquor, and the reaction liquor was neutralized to pH 8 with 62 % sulfuric acid (16 g) to precipitate a crystal. The crystal thus obtained was filtered by suction to separate the crystal, and the separated crystal was fully washed with water and further with methanol. The washed crystal was then dried, thus producing 13.1 g of 2-(2-hydroxy-5-t-octylphenyl)benzotriazole having a melting point of 103 to 105 ° C at the yield of 81.0 %.

Example 12

2-(2-hydroxy-3-t-butyl-5-methylphenyl)-5-chlorobenzotriazole-N-oxide (16.6 g) was added to a mixture of methanol (72 ml), water (20 ml) and 97% sodium hydroxide (12.4 g), and the resultant mixture was stirred at 70 ° C for 30 minutes, then cooled to 65 ° C. After adding 9-fluorenone (0.8 g) to the resultant mixture, 80 % paraformaldehyde (2.5 g) was added to the mixture for 30 minutes, and the resultant mixture was stirred at 60 to 70 ° C for 1 hour, and further at the boiling point (73 ° C for 4 hours; 2-(2-hydroxy-3-t-butyl-5-methylphenyl)-5-chlorobenzotriazole-N-oxide disappeared, to complete the reduction reaction.

Thereafter, water (50 ml) was added to the reaction liquor by dropwise at 65 to 70 ° C, and the reaction liquor was neutralized with 62.5 % sulfuric acid (12 g) to precipitate a crystal. The crystal thus obtained was filtered by suction to separate the crystal, and the separated crystal was fully washed with water and further with methanol. The washed crystal was then dried, thus producing 14.7 g of 2-(2-hydroxy-3-t-butyl-5-methylphenyl)-5-chlorobenzotriazole having a melting point of 138 to 140 ° C at the yield of 93.0 %.

Example 13

The same procedure as in Example 12 was repeated except that 2-(2-hydroxy-3-t-butyl-5-methyl-

phenyl)-5-chlorobenzotriazole-N-oxide (16.6 g) was replaced respectively by (a) 2-(2-hydroxy-3,5-di-t-butylphenyl)-5-chlorobenzotriazole-N-oxide (19.5 g), (b) 2-(2-hydroxy-3,5-di-t-butylphenyl)benzotriazole-N-oxide (17.8 g) and (c) 2-(2-hydroxy-3,5-di-t-amylphenyl)benzotriazole-N-oxide (19.2 g).

The products thus obtained and their properties are as follows:

(a) 2-(2-hydroxy-3,5-di-t-butylphenyl)-5-chlorobenzotriazole:
Yield: 16.1 g (90.0 %), Melting Point: 154 to 155.5 ° C

(b) 2-(2-hydroxy-3,5-di-t-butylphenyl)benzotriazole:
Yield: 14.8 g (91.5 %), Melting Point: 150 to 152 ° C

(c) 2-(2-hydroxy-3,5-di-t-amylphenyl)benzotriazole:
Yield: 15.4 g (88.0 %), Melting Point: 77 to 79 ° C

Example 14

2-(2-hydroxy-3-t-butyl-5-methylphenyl)-5-chlorobenzotriazole-N-oxide (16.6 g) was added to a mixture of ethyl alcohol (80 ml), water (10 ml) and 95% potassium hydroxide (17.7 g), and the resultant mixture was stirred at 80 ° C for 30 minutes, then cooled to 65 ° C. After adding anthrone (1.4 g) to the resultant mixture, 35 % formalin (6.5 g) was added to the mixture dropwise for 30 minutes, and the resultant mixture was stirred at 65 to 70 ° C for 1 hour, and further at the boiling point (87 ° C) for 10 hours; 2-(2-hydroxy-3-t-butyl-5-methylphenyl)-5-chlorobenzotriazole-N-oxide disappeared, to complete the reduction reaction.

Thereafter, water (50 ml) was slowly added to the reaction liquor, and the reaction liquor was neutralized with 62.5 % sulfuric acid (12 g) to precipitate a crystal. The crystal thus obtained was filtered by suction to separate the crystal, and the separated crystal was fully washed with water and further with ethyl alcohol. The washed crystal was then dried, thus producing 14.5 g of 2-(2-hydroxy-3-t-butyl-5-methylphenyl)-5-chlorobenzotriazole having a melting point of 138 to 140 ° C at the yield of 92.0 %.

Example 15

2-(2-hydroxy-3,5-di-t-butylphenyl)-5-chlorobenzotriazole-N-oxide (18.7 g) was added to a mixture of methanol (80 ml), water (30 ml) and 97% sodium hydroxide (12.4 g), and the resultant mixture was stirred at 70 ° C for 30 minutes, then cooled to 60 ° C. After adding benzanthrone (1.2 g) to the resultant mixture, 90 % acetoaldehyde (3.2 g) was added to the mixture dropwise for 30 minutes, and the resultant mixture was stirred at 60 to 70 ° C for 30 minutes, and further at the boiling point (74 ° C) for 4 hours; 2-(2-hydroxy-3,5-di-t-butylphenyl)-5-chlorobenzotriazole-N-oxide disappeared, to complete the reduction reaction.

Thereafter, water (50 ml) was added to the reaction liquor by dropwise at 65 to 70 ° C, and the reaction liquor was neutralized with 62.5 % sulfuric acid (12 g) to precipitate a crystal. The crystal thus obtained was filtered by suction to separate the crystal, and the separated crystal was fully washed with water and further with methanol. The washed crystal was then dried, thus producing 15.9 g of 2-(2-hydroxy-3,5-di-t-butylphenyl)-5-chlorobenzotriazole having a melting point of 154 to 155.5 ° C at the yield of 89.0 %.

Example 16

2-(2-hydroxy-5-methylphenyl)benzotriazole-N-oxide (10.7 g) was added to a mixture of methanol (60 ml), water (30 ml) and 97% sodium hydroxide (12.4 g), and the resultant mixture was heated to 70 ° C. After adding 9-fluorenone 1.0 g to the resultant mixture, 80 % paraformaldehyde 2.5 g was added to the mixture for 30 minutes, and the resultant mixture was heated to the boiling point (75 ° C). The resultant mixture was further stirred at the boiling point for 4 hours; 2-(2-hydroxy-5-methylphenyl)benzotriazole-N-oxide disappeared,to complete the reduction reaction.

Thereafter, water (50 ml) was added to the reaction liquor, and the reaction liquor was neutralized with 62 % sulfuric acid (16 g) to precipitate a crystal. The crystal thus obtained was filtered by suction to separate the crystal, and the separated crystal was fully washed with water and further with methanol. The washed crystal was then dried, thus producing 9.2 g of 2-(2-hydroxy-5-methylphenyl)benzotriazole having a melting point of 129 to 130 ° C at the yield of 92.1 %.

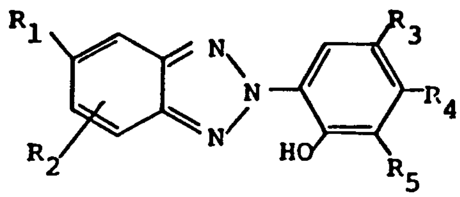

(I)

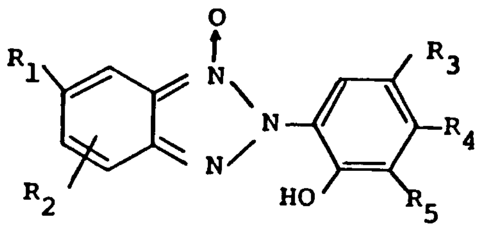

(II)

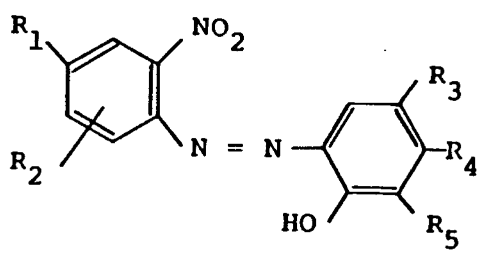

(III)

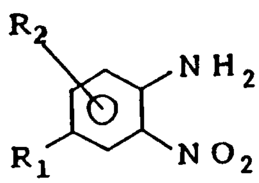

(IV)

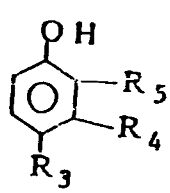

(V)

## Claims

1. A method for preparing a 2-phenylbenzotriazole of formula I

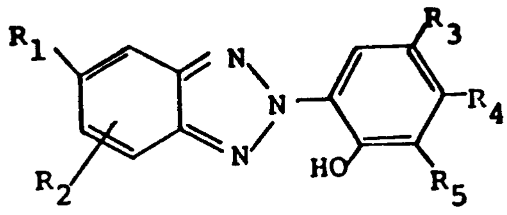

(I)

wherein $R_1$ is H, Cl, $C_{1-4}$ alkyl, $C_{1-4}$ alkoxy, COOH or $SO_3H$; $R_2$ is H, Cl, $C_{1-4}$ alkyl or $C_{1-4}$ alkoxy; $R_3$ is H, Cl, $C_{1-12}$ alkyl, $C_{1-4}$ alkoxy, phenyl, ($C_{1-8}$ alkyl)phenyl, phenoxy or phenyl ($C_{1-4}$ alkyl); $R_4$ is H, Cl, OH or $C_{1-4}$ alkoxy; and $R_5$ is H, $C_{1-12}$ alkyl or phenyl($C_{1-4}$ alkyl), which comprises reducing a nitroazobenzene of formula III

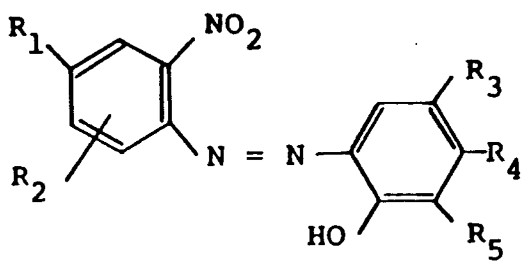

(III)

wherein $R_1$, $R_2$, $R_3$, $R_4$ and $R_5$ are as defined above, with an aldehyde, in the presence of base and an aromatic keytone selected from anthrone, benzanthrone and 9-fluorenone.

2. A method according to claim 1, wherein 2 to 4 mol aldehyde are used per mol nitroazobenzene (III).

3. A method according to claim 1, wherein, in the first of two steps, 1 to 2 mol aldehyde are used per mol nitroazobenzene (III), to give an intermediate which is a 2-phenylbenzotriazole-N-oxide of formula II

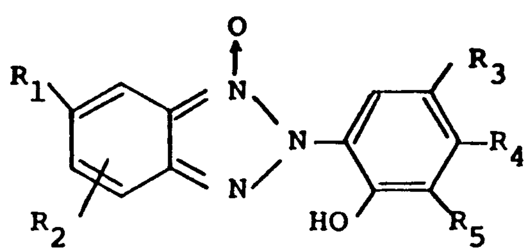

(II)

wherein $R_1$, $R_2$, $R_3$, $R_4$ and $R_5$ are as defined in claim 1, and, in the second step, 1 to 4 mol aldehyde are used per mol intermediate (II).

4. A method for preparing a 2-phenylbenzotriazole (I) as defined in claim 1, which comprises reducing 2-phenylbenzotriazole-N-oxide (II) as defined in claim 3 with, per mol thereof, 1 to 4 mol aldehyde, in the presence of base and an aromatic ketone as defined in claim 1.

5. A method for preparing a 2-phenylbenzotriazole-N oxide (II) as defined in claim 3, which comprises reducing nitroazobenzene (III) as defined in claim 1 with, per mol thereof, 1 to 2 mol aldehyde, in the presence of base and an aromatic ketone as defined in claim 1.

6. A method according to any preceding claim, wherein the aldehyde reducing agent is selected from formaldehyde, paraformaldehyde, acetaldehyde, benzaldehyde and anisaldehyde.

7. A method according to any preceding claim, wherein the aromatic ketone is used in an amount of 0.2 to 30% by weight, based on the weight of the starting material (II or III).

8. A method according to any preceding claim, wherein the base is sodium hydroxide or potassium hydroxide in an amount of 1 to 12 mol per mol starting material (II or III).

## Revendications

1. Procédé pour la préparation d'un 2-phénylbenzo-triazole de formule I

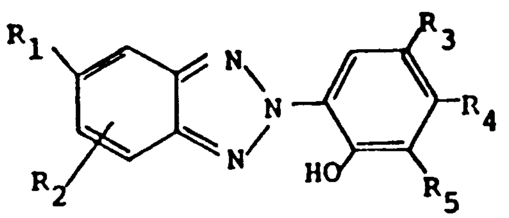

(I)

dans laquelle $R_1$ est H, Cl, un groupe alkyle en $C_{1-4}$, alcoxy en $C_{1-4}$, COOH ou $SO_3H$; $R_2$ est H, Cl, un groupe alkyle en $C_{1-4}$ ou alcoxy en $C_{1-4}$; $R_3$ est H, Cl, un groupe alkyle en $C_{1-12}$, alcoxy en $C_{1-4}$, phényle, alkyl($C_{1-8}$)-phényle, phénoxy ou phényl-alkyle($C_{1-4}$); $R_4$ est H, Cl, OH ou un groupe alcoxy en $C_{1-4}$; et $R_5$ est H, un groupe alkyle en $C_{1-12}$ ou phényl-alkyle($C_{1-4}$), comprenant la réduction d'un nitroazobenzène de formule III

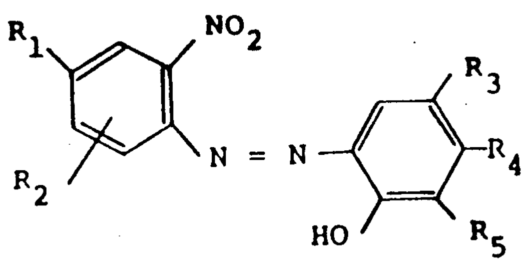

(III)

dans laquelle $R_1$, $R_2$, $R_3$, $R_4$ et $R_5$ sont tels que définis ci-dessus, à l'aide d'un aldéhyde, en présence d'une base et d'une cétone aromatique choisie parmi l'anthrone, la benzanthrone et la 9-fluorénone.

2. Procédé selon la revendication 1, dans lequel on utilise de 2 à 4 moles d'aldéhyde par mole de nitroazobenzène (III).

3. Procédé selon la revendication 1, dans lequel, dans la première de deux étapes, on utilise de 1 à 2 moles d'aldéhyde par mole de nitroazobenzène (III), pour obtenir un produit intermédiaire qui est un N-oxyde de 2-phénylbenzo-triazole de formule II

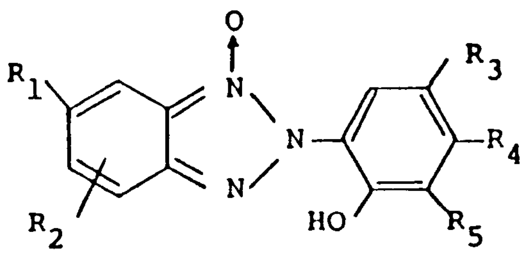

(II)

dans laquelle $R_1$, $R_2$, $R_3$, $R_4$ et $R_5$ sont tels que définis dans la revendication 1, et, dans la seconde étape, on utilise de 1 à 4 moles d'aldéhyde par mole de produit intermédiaire (II).

**4.** Procédé pour la préparation d'un 2-phénylbenzo-triazole (I) tel que défini dans la revendication 1, comprenant la réduction d'un N-oxyde de 2-phénylbenzotriazole (II), tel que défini dans la revendication 3, à l'aide, par mole de celui-ci, de 1 à 4 moles d'aldéhyde, en présence d'une base et d'une cétone aromatique telle que définie dans la revendication 1.

**5.** Procédé pour la préparation d'un N-oxyde de 2-phénylbenzotriazole (II) tel que défini dans la revendication 3, comprenant la réduction d'un nitroazobenzène (III) tel que défini dans la revendication 1, à l'aide, par mole de celui-ci, de 1 à 2 moles d'aldéhyde, en présence d'une base et d'une cétone aromatique telle que définie dans la revendication 1.

**6.** Procédé selon l'une quelconque des revendications précédentes, dans lequel le réducteur de type aldéhyde est choisi parmi le formaldéhyde, le paraformaldéhyde, l'acétaldéhyde, le benzaldéhyde et l'anisaldéhyde.

**7.** Procédé selon l'une quelconque des revendications précédentes, dans lequel on utilise la cétone aromatique en une quantité allant de 0,2 à 30 % en poids, par rapport au poids du produit de départ (II) ou (III).

**8.** Procédé selon l'une quelconque des revendications précédentes, dans lequel la base est l'hydroxyde de sodium ou l'hydroxyde de potassium en une quantité de 1 à 12 moles par mole du produit de départ (II) ou (III).

## Patentansprüche

**1.** Verfahren zum Herstellen eines 2-Phenylbenzotriazols der Formel I

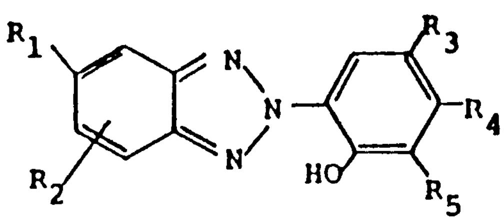

(I),

wobei $R_1$ die Bedeutung H, Cl, $C_{1-4}$-Alkyl, $C_{1-4}$-Alkoxy, COOH oder $SO_3H$ hat; $R_2$ die Bedeutung H, Cl, $C_{1-4}$-Alkyl oder $C_{1-4}$Alkoxy hat; $R_3$ die Bedeutung H, Cl, $C_{1-12}$-Alkyl, $C_{1-4}$-Alkoxy, Phenyl, ($C_{1-8}$-Alkyl)phenyl, Phenoxy oder Phenyl($C_{1-4}$-alkyl) hat; $R_4$ die Bedeutung H, Cl, OH oder $C_{1-4}$-Alkoxy hat; und $R_5$ die Bedeutung H, $C_{1-12}$-Alkyl oder Phenyl($C_{1-4}$-alkyl) hat, wobei man ein Nitroazobenzol der Formel III

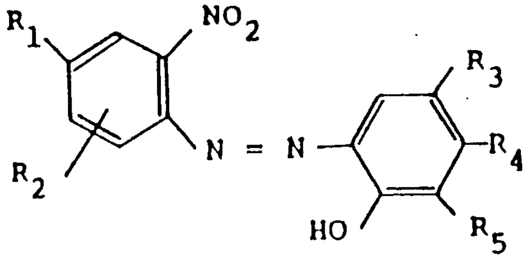

(III),

wobei $R_1$, $R_2$, $R_3$, $R_4$ und $R_5$ die vorstehend definierten Bedeutungen haben, mit Aldehyd in Gegenwart von Base und einem aromatischen Keton reduziert, welches ausgewählt wird aus Anthron, Benzanthron und 9-Fluorenon.

**2.** Verfahren nach Anspruch 1, wobei man pro Mol Nitroazobenzol(III) 2 bis 4 Mol Aldehyd verwendet.

**3.** Verfahren nach Anspruch 1, wobei man im ersten von zwei Schritten 1 bis 2 Mol Aldehyd pro Mol Nitroazobenzol (III) verwendet, um ein Zwischenprodukt in Gestalt eines 2-Phenylbenzotriazol-N-oxids der Formel II

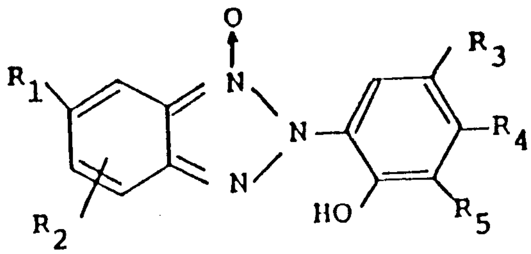

(II),

zu erhalten, wobei $R_1$, $R_2$, $R_3$, $R_4$ und $R_5$ die vorstehend in Anspruch 1 definierte Bedeutung haben und im zweiten Schritt 1 bis 4 Mol Aldehyd pro Mol Zwischenprodukt (II) verwendet.

**4.** Verfahren zum Herstellen eines Phenylbenzotriazols (I) wie in Anspruch 1 definiert, wobei man ein wie in Anspruch 3 definiertes 2-phenylbenzotriazol-N-oxid (II) in Anwesenheit von Base und eines wie in Anspruch 1 definierten aromatischen Ketons mit 1 bis 4 Mol Aldehyd pro Mol 2-Phenylbenzotriazol-N-oxid (II) reduziert.

**5.** Verfahren zum Herstellen eines Phenylbenzotriazol-N-oxids (11) wie in Anspruch 3 definiert, wobei man wie in Anspruch 1 definiertes Nitroazobenzol (III) in Gegenwart von Base und eines wie in Anspruch 1 definierten aromatischen Ketons mit 1 bis 2 Mol Aldehyd pro Mol Nitroazobenzol (III) reduziert.

**6.** Verfahren nach einem der vorhergehenden Ansprüche, wobei man das Aldehyd-Reduktionsmittel auswählt aus Formaldehyd, Paraformaldehyd, Acetaldehyd, Benzaldehyd und Anisaldehyd.

**7.** Verfahren nach einem der vorhergehenden Ansprüche, wobei man das aromatische Keton, bezogen auf das Gewicht des Ausgangsstoffs (II oder III), in einer Menge von 0,2 bis 30 Gew.-% einsetzt.

**8.** Verfahren nach einem der vorhergehenden Ansprüche, wobei die Base Natriumhydroxid oder Kaliumhydroxid in einer Menge von 1 bis 12 Mol pro Mol Ausgangsstoff (II oder III) ist.